# EUROPEAN PATENT SPECIFICATION

(11) **EP 2 255 775 B1**
(45) Date of publication and mention of the grant of the patent: **25.02.2015**
(21) Application number: 10004667.1
(22) Date of filing: 04.05.2010
(51) Int. Cl.: A61J 15/00, A61M 25/04, A61M 39/10

(54) **Connecting structure for a gastrostomy catheter and a fluid supply tube**
Verbindungsstruktur für einen Gastrostomiekatheter und einen Flüssigkeitszuleitungsschlauch
Connecteur entre un cathéter pour gastrostomie et un tube d'alimentation en fluide

(30) Priority: 28.05.2009 JP 2009128871
(43) Date of publication of application: 01.12.2010
(73) Proprietor: Covidien LP, Mansfield, MA 02048 (US)
(72) Inventor: Abe, Kazuhiro, Fukuroi Shizuoka 437-0004 (JP); Watanabe, Motonori, Fukuroi Shizuoka 437-0004 (JP)
(74) Representative: Tomlinson, Edward James

(56) References cited:
- WO-A1-2005/105017
- WO-A1-2008/003785

## Description

### [Technical Field]

The present invention relates to a connecting structure for a gastrostomy catheter and a fluid supply tube which are used for supplying a fluid such as nutrients or food in fluid form to the gastrointestinal tract of a patient.

### [Prior Art]

Fluids such as nutrients or food in fluid form are conventionally supplied to people having a reduced capacity for ingesting food orally by themselves due to advanced age or illness (referred to hereinafter as "patient(s)") using a gastrostomy catheter. The gastrostomy catheter generally comprises a tubular part which passes through a gastric fistula for ingestion which is formed in the patient's abdomen, an external holding part which is attached to the base end of the tubular part and placed on the surface of the skin at the abdomen, and an internal holding part which is attached to the tip end of the tubular part and is placed inside the gastrointestinal tract, in the stomach. See for instance WO2008/003785. This kind of gastrostomy catheter is fitted in a gastric fistula formed in the patient's abdomen, and the wall of the gastrointestinal tract and the abdomen wall are fixed, after which a fluid supply tube is connected and fluid is supplied to the gastrointestinal tract via this fluid supply tube.

If the fluid supply tube is pressed strongly into this kind of gastrostomy catheter when the fluid supply tube is connected to the gastrostomy catheter, there is a risk of the external holding part of the gastrostomy catheter becoming embedded in the gastric fistula inside the body. In order to prevent the external fixing part from becoming embedded in the gastric fistula inside the body, the fluid supply tube needs to be connected to the gastrostomy catheter without the fluid supply tube strongly pressing the gastrostomy catheter. For this reason, there is a component in the gastrostomy catheter which is provided so that the fluid supply tube is made to engage with the external holding part of the gastrostomy catheter from a direction perpendicular to the longitudinal direction of the gastrostomy catheter (see Patent Document 1, for example).

With this catheter for establishing a gastric fistula (gastrostomy catheter), a flange is formed at the top of an adapter (external holding part), and legs which can engage with the flange of the catheter for establishing a gastric fistula are provided at the tip end of a feeding tube assembly (fluid supply tube). The feeding tube assembly is designed to be connected in a state in which it is perpendicular to the longitudinal direction of the catheter for establishing a gastric fistula, and the tip-end opening thereof is formed to be perpendicular to the feeding tube of the feeding tube assembly. A space which is the thickness of the flange from the tip-end opening is then provided in order to provide for the legs. Moreover, the base end of the legs is coupled to the tip end of the feeding tube assembly.

### [Prior Art Document]

### [Patent Document]

[Patent Document 1] Published Japanese Translation of a PCT Application 2008-504896

### [Summary of the Invention]

However, with the gastrostomy catheter described above, the fluid passage of the gastrostomy catheter and the fluid passage of the fluid supply tube which is connected to the gastrostomy catheter are at right-angles, and therefore the flow of fluid from the fluid supply tube to the gastrostomy catheter is not smooth. Furthermore, there are problems in that the fluid is likely to leak at the connection between the gastrostomy catheter and the fluid supply tube.

The present invention has been devised in order to resolve the problems mentioned above, and it aims to provide a connecting structure for a gastrostomy catheter and a fluid supply tube with which the gastrostomy catheter does not press the body, the flow of fluid is smooth, and leakage is unlikely to occur.

In order to achieve the aim described above, structural features of the connecting structure for a gastrostomy catheter and a fluid supply tube lie in the fact that it is a connecting structure for connecting a gastrostomy catheter which is fitted in a gastric fistula formed between the abdominal wall and gastrointestinal tract of a patient, and a fluid supply tube which is connected to the gastrostomy catheter for supplying fluid to the patient's gastrointestinal tract through a fluid passage provided in the gastrostomy catheter; the section of the gastrostomy catheter which is disposed on the surface side of the abdominal wall at the gastric fistula comprises an external holding part having an opening into the fluid passage, and a guide part, of which the inner peripheral edge is substantially C-shaped or U-shaped when seen from above, is linked to the external holding part by a linking part, in a state in which it is spaced apart from the opening side of the external holding part; and a cylindrical engaging part is provided in the region of the tip end of a tube main body of the fluid supply tube, said engaging part being coaxial with the tube main body and able to engage between the external holding part and guide part in a state in which the tip end of the tube main body is placed in communication with the opening of the external holding part by making the tube main body enter the guide part from the open side thereof so that it advances between the external holding part and the guide part.

With the connecting structure for a gastrostomy catheter and a fluid supply tube according to the present invention, a linking part extends from the external holding part of the gastrostomy catheter, and a guide part which is substantially C-shaped or U-shaped when seen from above is linked to the linking part. In this case, the inner space of the substantially C-shaped or U-shaped guide part is positioned on the line of extension of the fluid passage, spaced apart from the external holding part. A cylindrical engaging part is then provided coaxially with the tube main body towards the tip end of the tube main body of the fluid supply tube, and this engaging part is designed to make the tip end of the tube main body communicate with the opening of the external holding part by introducing the tube main body into the guide part from the open side thereof, in other words, by making the tube main body advance and engaging between the external holding part and the guide part from a direction which is substantially perpendicular to the fluid passage of the gastrostomy catheter.

Accordingly, the fluid supply tube is connected to the gastrostomy catheter by moving the engaging part in such a way that it slides laterally between the external holding part and the guide part, and there is no need to press the fluid supply tube into the gastrostomy catheter. This means that when the fluid supply tube is connected to the gastrostomy catheter, the external holding part does not press on the gastric fistula inside the body and does not become embedded. Furthermore, the engaging part is provided coaxially with the tube main body, and therefore when the fluid supply tube has been connected to the gastrostomy catheter, the fluid passage of the gastrostomy catheter and the fluid passage of the fluid supply tube are joined substantially linearly. Consequently, there are no problems in terms of the fluid not readily flowing from the fluid supply tube to the gastrostomy catheter, or in terms of fluid being likely to leak at the connection between the gastrostomy catheter and the fluid supply tube. Moreover, the inner peripheral edge of the guide part should be substantially C-shaped or U-shaped, but the outer peripheral edge need not be substantially C-shaped or U-shaped.

Further structural features of the connecting structure for a gastrostomy catheter and a fluid supply tube lie in the fact that the guide part consists of an elastic body, and a raised part is formed at the peripheral edge of the opening in the external holding part; the length between the section of the engaging part which is in contact with the guide part and the tip end of the tube main body is greater than the length between the raised part and the guide part, and when the engaging part and the section of the tube main body protruding from the engaging part which runs from a section in the region of the tip end of the tube main body to the tip end thereof are pushed between the raised part and the guide part so that the tip end of the tube main body is placed in communication with the opening of the external holding part, the engaging part is designed to be able to engage between the raised part and the guide part.

This means that the operation to make the engaging part engage between the external holding part and the guide part is uncomplicated because it simply involves pushing the engaging part and the section of the tube main body protruding from the engaging part which runs from a section in the region of the tip end of the tube main body to the tip end thereof between the raised part and the guide part. In this case, the guide part returns to its original state after it has been urged to bend in such a way that the space formed with the raised part widens. In addition, in accordance with the present invention, the structures of the engaging part and the guide part can be simplified.

Further structural features of the connecting structure for a gastrostomy catheter and a fluid supply tube lie in the fact that the raised part is formed at the peripheral edge of the opening in the external holding part, and the length between the section of the engaging part which is in contact with the guide part and the tip end of the tube main body is greater than the length between the raised part and the guide part; and provision is made for a deforming mechanism which allows deformation under pressure at the raised part so that the length between the raised part and the guide part becomes greater, or provision is made for a deforming mechanism which allows deformation under pressure at the tip end of the tube main body so that the length between the section of the engaging part which is in contact with the guide part and the tip end of the tube main body becomes shorter.

The deforming mechanism in this case may comprise a soft material which is depressed and contracted by pressure on the raised part, or it may comprise a mechanism wherein a specific section advances or retracts by the application of pressure or the release of pressure. Furthermore, the deforming mechanism may comprise a soft material which is depressed and contracted by pressure on the tip end of the tube main body, or it may comprise a mechanism wherein a specific section advances or retracts by the application of pressure or the release of pressure. That is to say, the deformation in this case involves depression and contraction etc., and the displacement involves a changing of position etc., in addition to the depression and contraction. This means that the operation to make the engaging part engage between the external holding part and the guide part is simplified.

Further structural features of the connecting structure for a gastrostomy catheter and a fluid supply tube lie in the fact that the deforming mechanism is configured by providing a spring mechanism inside the engaging part, for urging the tip end of the tube main body outwards with respect to the engaging part, and the tip end of the tube main body is caused to retract inside the engaging part by the application of pressure thereto.

In this case, the engaging part can engage between the external holding part and the guide part by holding the engaging part in the hand and pushing it between the external holding part and the guide part, and in this process, the tip end of the tube main body is pressed by the raised part so as to retract inside the engaging part. This means that the operation to make the engaging part engage between the external holding part and the guide part and the operation to release this engagement can be carried out more reliably. Moreover, the tip end of the tube main body in this case is made of a rigid material and even if the spring contracts under pressure, the tip end of the tube main body preferably does not contract and deform, or it only contracts and deforms by a smaller amount than the contraction of the spring.

Further structural features of the connecting structure for a gastrostomy catheter and a fluid supply tube lie in the fact that the guide part consists of an elastic body, and respective protrusions are formed at both ends of the guide part on the faces opposite the external holding part; and when the engaging part is made to engage between the external holding part and the guide part, the guide part bends so that the protrusions go past the end face of the engaging part, after which these protrusions engage at the side of the engaging part. This means that the engagement between the engaging part and the guide part is more secure.

Further structural features of the connecting structure for a gastrostomy catheter and a fluid supply tube lie in the fact that the guide part and the linking part are constituted by a connecting member which is separate from the external holding part, and a circular fitting groove is formed in the circumferential direction of the external holding part on the outer peripheral surface of the external holding part; and a fitting part which is rotatably fitted in the circular fitting groove is included in the linking part. This means that the connecting member can rotate in the axial direction with respect to the gastrostomy catheter, and therefore cleaning is simplified when the external holding part of the gastrostomy catheter is soiled, or the like.

Further structural features of the connecting structure for a gastrostomy catheter and a fluid supply tube lie in the fact that the gastrostomy catheter comprises: the external holding part; a tubular part which is disposed in such a way that the base end thereof is joined to the external holding part and the tip end section thereof extends from the gastric fistula into the gastrointestinal tract; and an internal holding part which is joined to the tip end of the tubular part and is disposed inside the gastrointestinal tract. This means that the gastrostomy catheter is not only properly attached inside the patient's body, but also outside the patient's body.

### [Brief Description of the Figures]

[Figure 1] is a front view showing a gastrostomy catheter and a fluid supply tube when they are connected by the connecting structure for a gastrostomy catheter and a fluid supply tube according to a first mode of embodiment of the present invention;
[Figure 2] is a front view showing the state before the gastrostomy catheter and fluid supply tube shown in Figure 1 are connected;
[Figure 3] shows the gastrostomy catheter, where (a) is a plan view, and (b) is a front view;
[Figure 4] is a bottom view showing the connecting member;
[Figure 5] is a bottom view showing the connecting member of a second mode of embodiment; and
[Figure 6] is a view in cross section showing the engaging part of a third mode of embodiment.

### [Modes of Embodiment of the Invention]

### (First Mode of Embodiment)

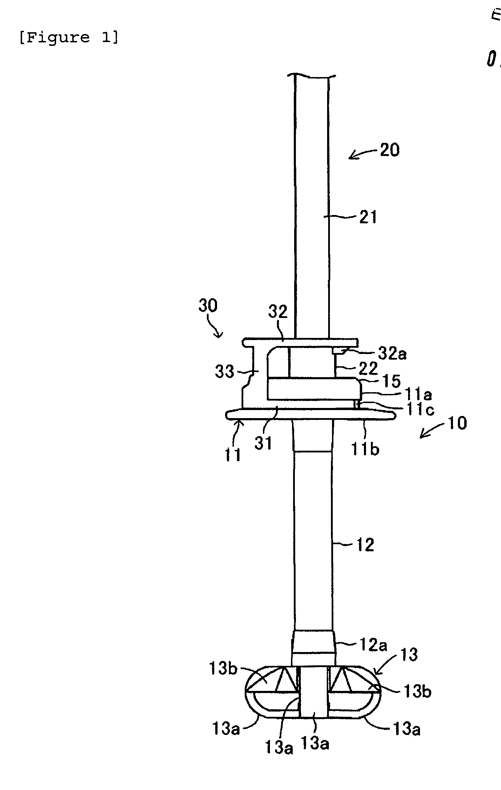

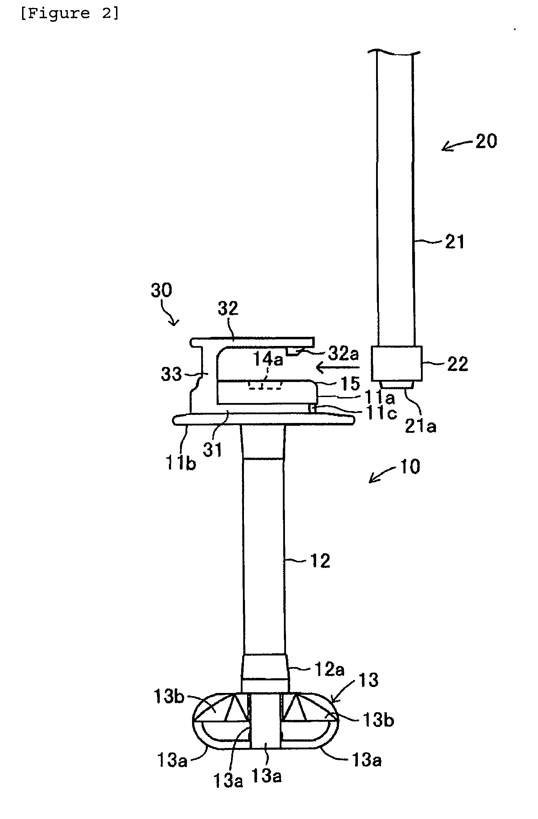

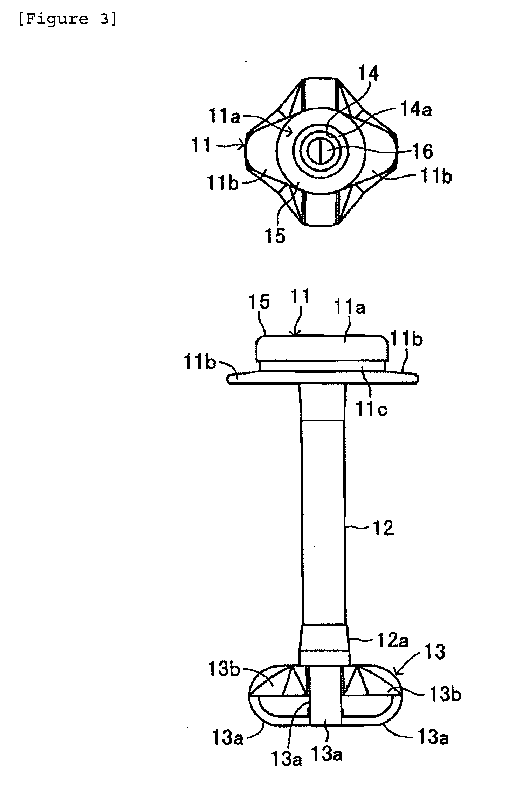

The first mode of embodiment of the present invention will be described below with the aid of the figures. Figures 1 and 2 show a gastrostomy catheter 10, fluid supply channel 20 and connecting member 30 which make up the connecting structure for a gastrostomy catheter and a fluid supply tube, in accordance with this mode of embodiment. As shown in Figure 3, the gastrostomy catheter 10 comprises an external holding part 11, a tubular part 12 which is joined at the centre on the lower surface of the external holding part 11, and an internal holding part 13 which is joined to the tip end of the tubular part 12. In the description that follows, the external holding part 11 side shall be referred to as the upper side/top/above, and the internal holding part 13 side shall be referred to as the lower side/bottom/below.

The external holding part 11 comprises an insertion opening 11a which is formed as a fairly thick annular shape, and a pair of protrusions 11b which project outwards from both sides at the lower end on the outer periphery of the insertion opening 11a. The protrusions 11b have the function of preventing the gastrostomy catheter 10 from becoming embedded inside the patient's body. Furthermore, a fitting groove 11c for the attachment of the connecting member 30 is formed in the peripheral direction on the upper side of the protrusions 11b, at the outer peripheral surface of the insertion opening 11a.

An insertion hole 14 running vertically is then formed at the centre of the insertion opening 11a, and the diameter of an upper opening 14a of the insertion hole 14 becomes steadily greater from the bottom towards the top. Furthermore, the diameter of the top part on the outer periphery of the insertion opening 11a becomes steadily smaller from the bottom towards the top, and by means of this the centre of the top part on the outer periphery of the insertion opening 11a is formed as a raised curved face. By forming the tapered upper opening 14a and the curved face at the top part on the outer periphery, an annular raised part 15 is formed at the upper face of the insertion opening 11a. A check valve 16 which has a slit formed in the centre is then provided on the inner peripheral surface of the insertion hole 14.

The tubular part 12 consists of an elongate cylindrical body inside which is formed a fluid passage (not depicted) for the passage of fluids such as nutrients and food in fluid form, and the upper end of the fluid passage communicates with the insertion hole 14 of the external holding part 11. The internal holding part 13 is connected to the tubular part 12 by way of a connector 12a which is fixed to the lower end of the tubular part 12. The internal holding part 13 comprises four strip-shaped linking parts 13a which extend in four directions from the edge of the opening at the lower end of the connector 12a, and four linking film parts 13b which are provided between the upper parts of each of the linking parts 13a, and the tip ends of the linking parts 13a are linked to one another.

The fluid supply tube 20 comprises a tube main body 21 and an engaging part 22 which is attached towards the lower end of the tube main body 21. The tube main body 21 consists of a soft tube having a fluid passage which communicates with the fluid passage of the tubular part 12, and it is substantially the same thickness as the tubular part 12. The tip end 21a of the tubular main body 21 enters the engaging part 22 by contracting under pressure in the axial direction. Furthermore, the tip end 21a can be fitted in a liquid-tight state with the upper section of the external holding part 11 including the upper opening 14a of the insertion hole 14, and when the tip end is fitted into the insertion hole 14, the slit of the check valve 16 is opened. This allows fluid to flow from the fluid passage of the tube main body 21 to the fluid passage of the gastrostomy catheter 10. The engaging part 22 consists of a cylindrical body made of a rigid resin material, and it is fixed to the outer periphery at the lower end of the tube main body 21 with the tip end 21a of the tube main body 21 able to be received therein.

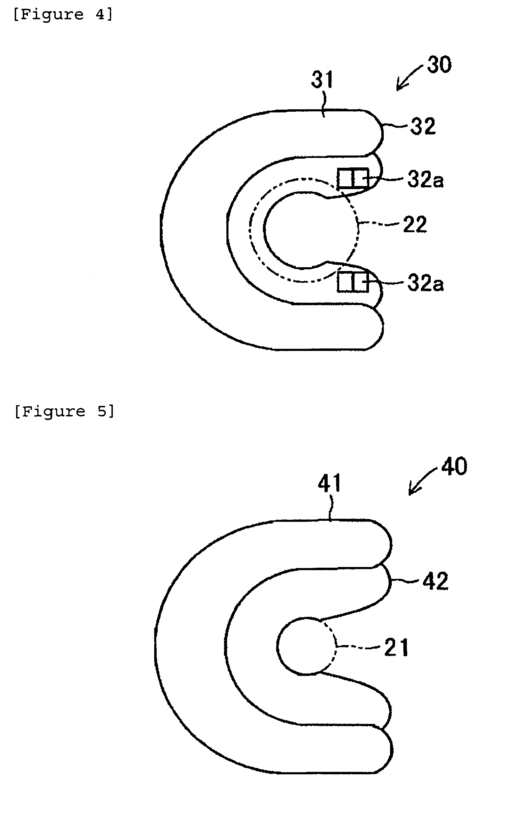

The connecting member 30 comprises a fitting part 31 which fits into the fitting groove 11c, a guide part 32 which is disposed parallel to and spaced apart from the fitting part 31, and a linking part main body 33 which links the fitting part 31 and the guide part 32. It should be noted that the linking part according to the present invention consists of the fitting part 31 and the linking part main body 33. Figure 4 shows the connecting member 30 seen from the bottom, and the fitting part 31 consists of a substantially U-shaped plate. The inside section on the substantially U-shaped inner peripheral edge of the fitting part 31 fits into the fitting groove 11c, whereby the connecting member 30 is rotatably assembled with the external holding member 11 of the gastrostomy catheter 10. The guide part 32 consists of a substantially C-shaped plate when seen from above (not depicted), and it is arranged parallel to the fitting part 31 and spaced apart from the fitting part 31.

The space between the fitting part 31 and the guide part 32 is set so that the space between the raised part 15 of the external holding part 11 and the guide part 32 is substantially equal to the length of the engaging part 22 in the axial direction when the fitting part 31 has been fitted into the fitting groove 11c. Furthermore, the inner peripheral edge of the guide part 32 is formed in such a way that that the width between opposing sections on the open side (the right-hand section in Figure 4) becomes steadily narrower moving from the open side to the inside, and the inside thereof is formed to become substantially circular (C-shaped). Respective protrusions 32a are then formed at both ends on the lower surface of the guide part 32.

When the lower surfaces of these protrusions 32a are seen from the side, the portions lying on the open side of the guide part 32 are formed as tapers which are oriented steadily downwards moving from the open side to the inside of the guide part 32, and the portions lying on the inside of the guide part 32 are formed to be horizontal. Furthermore, the spacing of the pair of protrusions 32a is somewhat narrower than the outer diameter of the engaging part 22. As shown by the two-dot chain line in Figure 4, when the engaging part 22 has engaged with the guide part 32, the pair of protrusions 32a are in contact with the outer peripheral surface of the engaging part 22, and the engaging part 22 is prevented from being removed from the guide part 32. The linking part main body 33 links the central part of the top face of the fitting part 31 and the central part of the bottom face of the guide part 32, and it consists of a plate-like linking piece which has an arcuate shape in cross section (not depicted).

With this configuration, when the fluid supply tube 20 is connected to the gastrostomy catheter 10 which has been fitted to the gastric fistula (not depicted) in the patient, the fitting part 31 is first of all fitted into the fitting groove 11c of the external fitting part 11 to assemble the connecting member 30 with the gastrostomy catheter 10. In this case, the two ends on the open side of the fitting part 31 are pushed into the two sides of the fitting groove 11c, and the connecting member 30 slides laterally with respect to the gastrostomy catheter 10. By means of this, the inside section on the inner peripheral edge of the fitting part 31 fits into the semicircular portion of the fitting groove 11c, and the connecting member 30 is assembled in a state in which it can rotate in the axial direction with respect to the gastrostomy catheter 10.

Next, as shown in Figure 2, the engaging part 22 is positioned in the region between the open side section of the guide part 32 of the connecting member 30 and the raised part 15 of the gastrostomy catheter 10, and the fluid supply tube 20 is brought closer to the gastrostomy catheter 10. Then, as shown by the arrow, the fluid supply tube 20 is moved towards the gastrostomy catheter 10, and the engaging part 22 is pushed between the raised part 15 and the guide part 32. At this point, the tip end 21a of the tube main body 21 is pressed by the raised part 15 and is received inside the engaging part 22, and the engaging part 22 enters between the raised part 15 and the guide part 32 while the upper ends on both sides of the engaging part 22 (the two sides at the front and rear in Figure 2) are pushed up against the tapered faces of the protrusions 32a.

At this point, the open side portion of the guide part 32 is bent upwards. Then, when the upper ends on both sides of the engaging part 22 have gone past the protrusions 32a, the tip end 21a of the tube main body 21 protrudes from the engaging part 22 so as to fit into the top section of the insertion hole 14 of the external holding part 11, and the state shown in Figure 1 is achieved. In this process, the tip end 21a of the tube main body 21 is in liquid-tight contact with the upper opening 14a, and the slit in the check valve 16 is opened. Furthermore, the guide part 32 returns to its original state and the protrusions 32a engage at the outer peripheral surface of the engaging part 22, as shown in Figure 4. By means of this, the engaging part 22 is prevented from being removed from the gastrostomy catheter 10.

In this state, fluid enters the tube main body 21 from the base end opening of the tube main body 21. Note that a supply apparatus housing the fluid has already been connected to the base end opening of the tube main body 21, and the fluid is supplied from the supply apparatus. As a result, the fluid is supplied from the fluid passage of the tube main body 21, through the insertion hole 14 and fluid passage of the tubular part 12, and into the patient's gastrointestinal tract, for example into the stomach. Furthermore, if fluid or soiling adheres to the external holding part 11 of the gastrostomy catheter 10, the fluid or soiling is wiped off using a specific cloth or paper. In this case, the connecting member 30 is turned to allow the whole surface of the external holding member 11 to be wiped.

As described above, with the connecting structure for a gastrostomy catheter and a fluid supply tube according to the present invention, the connecting member 30 provided with the guide part 32 is attached to the external holding part 11 of the gastrostomy catheter 10. The engaging part 22 is then provided towards the tip end of the tube main body 21 of the fluid supply tube 20, and this engaging part 22 is pushed between the external holding part 11 and the guide part 32 so as to engage, whereby the tip end 21a of the tube main body 21 is placed in communication with the upper opening 14a of the external holding part 11.

Accordingly, the fluid supply tube 20 is connected to the gastrostomy catheter 10 by pushing the engaging part 22 laterally between the external holding part 11 and the guide part 32, and there is no need to press the fluid supply tube 20 into the gastrostomy catheter 10 from above. Thanks to this, the external holding part 11 does not become embedded by being pressed inside the body. Furthermore, when the fluid supply tube 20 has been connected to the gastrostomy catheter 10, the fluid passage of the gastrostomy catheter 10 and the fluid passage of the fluid supply tube 20 are joined substantially linearly, and therefore the fluid is not obstructed as it flows from the fluid supply tube 20 to the gastrostomy catheter 10, and leakage of the fluid at the connection between the gastrostomy catheter 10 and the fluid supply tube 20 is not likely to occur.

Furthermore, the tip end 21a of the tube main body 21 is made of a soft material, so when the tip end 21a is pressed, the tip end 21a contracts and is received inside the engaging part 22, and therefore the operation to make the engaging part 22 engage between the external holding part 11 and the guide part 32 is simplified. In addition, the respective protrusions 32a are formed at the lower surface at both ends of the guide part 32 so that when the engaging part 22 has engaged between the external holding part 11 and the guide part 32, the protrusions 32a are designed to engage on the side of the engaging part 22, and therefore the engagement between the engaging part 22 and the guide part 32 is more secure. Furthermore, the connecting member 30 is able to rotate with respect to the external holding part 11, and therefore cleaning is simpler when the external holding part 11 becomes soiled, or similar. In addition, the internal holding part 13 is provided at the lower part of the gastrostomy catheter 10, and therefore the gastrostomy catheter 10 is prevented from being easily withdrawn from the body when the gastrostomy catheter 10 is pulled outwards from the body.

### (Second Mode of Embodiment)

Figure 5 shows a connecting member 40 of the connecting structure for a gastrostomy catheter and a fluid supply tube according to a second mode of embodiment of the present invention. This connecting member 40 comprises a fitting part 41 which fits into the fitting groove 11c, a guide part 42 which is arranged parallel to and spaced apart from the fitting part 41, and a linking part main body (not depicted) which links the fitting part 41 and the guide part 42. The fitting part 41 consists of a substantially U-shaped plate having the same shape as the fitting part 31 described above. The guide part 42 consists of a substantially C-shaped plate when seen from above (not depicted), and it is disposed parallel to the fitting part 41 and spaced apart from the fitting part 41.

Furthermore, the inner peripheral edge of the guide part 42 is formed in such a way that that the width between opposing sections on the open side (the right-hand section in Figure 5) becomes steadily narrower moving from the open side to the inside, and the inside thereof is formed to become substantially circular (C-shaped), like the inner peripheral edge of the guide part 32 described above, but the substantially circular section on the inside is smaller than the circular section of the guide part 32. When the engaging part 22 has been assembled with the connecting member 40, the tube main body 21 is pushed in and engages with the circular section on the inside of the inner peripheral edge of the guide part 42, as shown by the two-dot chain line in Figure 5, and the fluid supply tube 20 is prevented from becoming removed from the gastrostomy catheter 10 and the connecting member 40. Note that this guide part 42 is not provided with protrusions.

The structures of the other components of the connecting structure for a gastrostomy catheter and a fluid supply tube according to this mode of embodiment are the same as those of the connecting structure for a gastrostomy catheter and a fluid supply tube according to the first mode of embodiment described above. Furthermore, with the connecting structure for a gastrostomy catheter and a fluid supply tube according to this mode of embodiment, the engaging part 22 engages between the external holding part 11 and the guide part 32, and the tube main body 21 engages within the inner peripheral edge of the guide part 42. The other operational effects of the connecting structure for a gastrostomy catheter and a fluid supply tube according to this mode of embodiment are the same as those of the connecting structure for a gastrostomy catheter and a fluid supply tube according to the first mode of embodiment described above.

### (Third Mode of Embodiment)

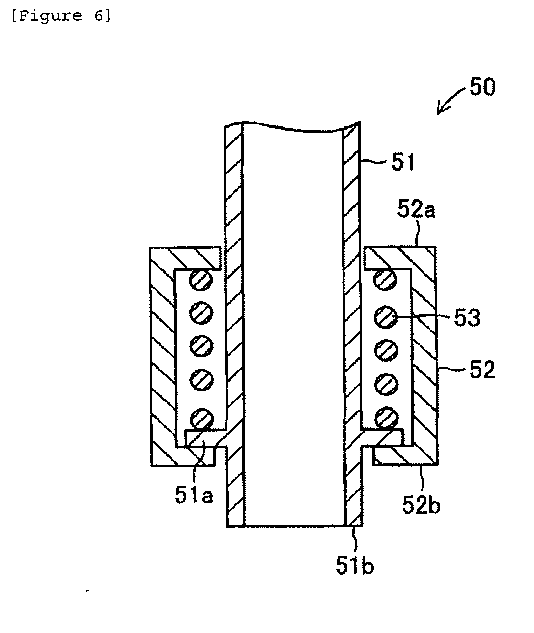

Figure 6 shows the main parts of a fluid supply tube 50 of the connecting structure for a gastrostomy catheter and a fluid supply tube according to a third mode of embodiment of the present invention. With this fluid supply tube 50, the lower end of the soft tube main body comprises a rigid supporting cylinder 51 for supporting an engaging part 52. This supporting cylinder 51 is joined to the upper portion of the tube main body by solvent-welding, and a flange-like spring-receiving part 51a is formed at the outer periphery of the lower section of the supporting cylinder 51. The engaging part 52 is then fitted to the spring-receiving part 51a with a spring 53 interposed. The engaging part 52 is provided with a ceiling part 52a and a bottom surface part 52b which lie at the top and bottom, respectively, of the cylindrical main body section, and are formed with a hole for the passage of the tube main body 51 therein.

The bottom surface part 52b is positioned below the spring-receiving part 51a, and the spring 53 is positioned between the ceiling part 52a and the spring-receiving part 51a, and the engaging part 52 is assembled with the supporting cylinder 51 in this way. The engaging part 52 is urged upwards by the spring-receiving part 51a and the spring 53. Consequently, when the tip end 51b of the supporting cylinder 51 is pressed upwards with respect to the engaging part 52, the spring 53 contracts and the tip end 51b is received inside the engaging part 52. The structures of the other components of the connecting structure for a gastrostomy catheter and a fluid supply tube according to this mode of embodiment are the same as those of the connecting structure for a gastrostomy catheter and a fluid supply tube according to the first mode of embodiment described above.

Furthermore, with the connecting structure for a gastrostomy catheter and a fluid supply tube according to this mode of embodiment, when the tip end 51b of the supporting cylinder 51 is pressed, the spring 53 contracts and the tip end 51b is received inside the engaging part 52, and therefore the operation to make the engaging part 52 engage between the external holding part 11 and the guide part 32, and to release the engagement thereof is simplified. The other operational effects of the connecting structure for a gastrostomy catheter and a fluid supply tube according to this mode of embodiment are the same as those of the connecting structure for a gastrostomy catheter and a fluid supply tube according to the first mode of embodiment described above.

Furthermore, as another mode of embodiment, the raised part 15 of the external holding part 11 of all of the modes of embodiment described above may be made of a soft material, and when the fluid supply tube 20 etc. is connected to the gastrostomy catheter 10, the tip end 21a etc. of the tube main body 21 etc. may be depressed by pressure on the raised part 15. This makes it possible to obtain the same operational effects as in each of the modes of embodiment described above.

The connecting structure for a gastrostomy catheter and a fluid supply tube according to the present invention is not limited to the modes of embodiment described above, and suitable modifications may be made within the technical scope of the present invention. For example, in the modes of embodiment described above, the connecting members 30, 40 are members which are separate from the external holding part 11, but the connecting members 30, 40 may be formed as a single piece with the external holding part 11. Furthermore, in the modes of embodiment described above, the connecting member 30 is able to rotate in the axial direction with respect to the gastrostomy catheter 10, but the fitting groove 11c may consist of two grooves which are parallel rather than circular, and the connecting member 30 may not be able to rotate. In addition, the internal holding part of the gastrostomy catheter may be a balloon-shaped (dome-shaped) part. Furthermore, it is possible to use an assembly which does not have the internal holding part for the gastrostomy catheter. In addition, a concertina-type spring, or similar, can be used instead of the coil spring 53 in the third mode of embodiment.

### [Key to Symbols]

10...gastrostomy catheter; 11...external holding part; 11c...fitting groove; 12...tubular part; 13...internal holding part; 14...insertion hole; 14a...upper opening; 15...raised part; 20, 50...fluid supply tube; 21...tube main body; 21a, 51b...tip end; 22, 52...engaging part; 30, 40...connecting member; 31, 41...fitting part; 32, 42...guide part; 32a...protrusion; 33...linking part main body.

## Claims

1. Gastrostomy catheter connecting structure for connecting a gastrostomy catheter which is fitted in a gastric fistula formed between the abdominal wall and gastrointestinal tract of a patient, the connecting structure comprising a gastrostomy catheter (10), a connecting member (30) and a fluid supply tube (20) for connection to the gastrostomy catheter for supplying fluid to the patient's gastrointestinal tract through a fluid passage provided in the gastrostomy catheter, said connecting structure being **characterized in that** the section of the gastrostomy catheter which in use is disposed on the surface side of the abdominal wall at the gastric fistula comprises an external holding part (11) having an opening (11a) into the fluid passage, the connecting member comprises a guide part (32), of which the inner peripheral edge is substantially C-shaped or U-shaped when seen from above, linked to the external holding part by a linking part (33), in a state in which the guide part is spaced apart from the opening side of the external holding part; and a cylindrical engaging part (22) is provided in the region of the tip end of a tube main body of the fluid supply tube, said engaging part being coaxial with the tube main body and able to engage between the external holding part and guide part in a state in which the tip end of the tube main body is placed in communication with the opening of the external holding part by introducing the tube main body into the guide part from the open side thereof so that it advances between the external holding part and the guide part.

2. Connecting structure according to Claim 1, in which the guide part consists of an elastic body, and a raised part (15) is formed at the peripheral edge of the opening in the external holding part; the length between the section (52a) of the engaging part which is in contact with the guide part and the tip end (21 a) of the tube main body is greater than the length between the raised part and the guide part, and when the engaging part and the section of the tube main body protruding from the engaging part which runs from a section in the region of the tip end of the tube main body to the tip end thereof are pushed between the raised part and the guide part so that the tip end of the tube main body is placed in communication with the opening of the external holding part, the engaging part is designed to be able to engage between the raised part and the guide part.

3. Connecting structure according to Claim 1 or 2, in which the raised part (15) is formed at the peripheral edge of the opening in the external holding part, and the length between the section (52a) of the engaging part which is in contact with the guide part and the tip end (21 a) of the tube main body is greater than the length between the raised part and the guide part; and provision is made for a deforming mechanism which allows deformation under pressure at the raised part so that the length between the raised part and the guide part becomes greater, or provision is made for a deforming mechanism which allows deformation under pressure at the tip end of the tube main body so that the length between the section of the engaging part which is in contact with the guide part and the tip end of the tube main body becomes shorter.

4. Connecting structure according to Claim 3, in which the deforming mechanism is configured by providing a spring mechanism (53) inside the engaging part, for urging the tip end of the tube main body outwards with respect to the engaging part, and the tip end of the tube main body is caused to retract inside the engaging part by the application of pressure thereto.

5. Connecting structure according to any one of Claims 1 to 4, in which the guide part consists of an elastic body, and respective protrusions (32a) are formed at both ends of the guide part on the faces opposite the external holding part; and when the engaging part is made to engage between the external holding part and the guide part, the guide part bends so that the protrusions go past the end face of the engaging part, after which these protrusions engage at the side of the engaging part.

6. Connecting structure according to any one of Claims 1 to 5, in which the guide part and the linking part are constituted by a connecting member which is separate from the external holding part, and a circular fitting groove (11c) is formed in the circumferential direction of the external holding part on the outer peripheral surface of the external holding part; and a fitting part (31) which is rotatably fitted in the circular fitting groove is included in the linking part.

7. Connecting structure according to any one of Claims 1 to 6, in which the gastrostomy catheter comprises: the external holding part; a tubular part (12) which is disposed in such a way that the base end thereof is joined to the external holding part and in use the tip end section thereof extends from the gastric fistula into the gastrointestinal tract; and an internal holding part (13) which is joined to the tip end of the tubular part and in use is disposed inside the gastrointestinal tract.

8. Connecting structure according to any preceding claim wherein said engaging part (22) is able to engage between the external holding part (11) and guide part (32) in a state in which the tip end (21 a) of the tube main body (21) is placed in communication with the opening (14a) of the external holding part by introducing the tube main body into the guide part from the open side thereof so that it advances between the external holding part and the guide part from a direction which is substantially perpendicular to a fluid passage direction of the gastrostomy catheter.

## Patentansprüche

1. Verbindungsstruktur für einen Gastrostomiekatheter zur Verbindung eines Gastrostomiekatheters, der in einer zwischen der Bauchwand und dem Magen-Darm-Trakt eines Patienten geformten Magenfistel angebracht ist, wobei die Verbindungsstruktur einen Gastrostomiekatheter (10), ein Verbindungsglied (30) und einen Flüssigkeitszuführungsschlauch (20) zur Verbindung mit dem Gastrostomiekatheter zur Zuführung von Flüssigkeit in den Magen-Darm-Trakt des Patienten durch einen in dem Gastrostomiekatheter bereitgestellten Flüssigkeitsdurchgang umfasst, wobei die Verbindungsstruktur **dadurch gekennzeichnet ist, dass** der Abschnitt des Gastrostomiekatheters, der im Gebrauch auf der Oberflächenseite der Bauchwand an der Magenfistel angeordnet ist, einen äußeren Halteteil (11) mit einer Öffnung (11a) in den Flüssigkeitsdurchgang umfasst, das Verbindungsglied einen Führungsteil (32) umfasst, dessen inneren Umfangsrand von oben gesehen im Wesentlichen C-förmig oder U-förmig ist, mit dem äußeren Halteteil über einen Verbindungsteil (33) verbunden ist, in einem Zustand, in dem der Führungsteil im Abstand von der Öffnungsseite des äußeren Halteteils angeordnet ist, und ein zylindrischer Eingriffsteil (22) in der Region des Spitzenendes eines Schlauchhauptkörpers des Flüssigkeitszuführungsschlauchs bereitgestellt ist, wobei der Eingriffsteil mit dem Schlauchhauptkörper koaxial ist und zum Eingriff zwischen dem äußeren Halteteil und dem Führungsteil in einem Zustand befähigt ist, in dem das Spitzenende des Schlauchhauptkörpers in Verbindung mit der Öffnung des äußeren Halteteils gesetzt wird, in dem der Schlauchhauptkörper in den Führungsteil von der offenen Seite her eingeführt wird, so dass er sich zwischen dem äußeren Halteteil und dem Führungsteil vorschiebt.

2. Verbindungsstruktur nach Anspruch 1, worin der Führungsteil aus einem elastischen Körper besteht und ein erhabener Teil (15) an dem Umfangsrand der Öffnung in dem äußeren Halteteil geformt ist; wobei die Länge zwischen dem Abschnitt (52a) des Eingriffsteils, der mit dem Führungsteil in Kontakt ist, und dem Spitzenende (21a) des Schlauchhauptkörpers größer als die Länge zwischen dem erhabenen Teil und dem Führungsteil ist, und wenn der Eingriffsteil und der Abschnitt des Schlauchhauptkörpers, der von dem Eingriffsteil vorsteht, der von einem Abschnitt in der Region des Spitzenendes des Schlauchhauptkörpers zu dem Spitzenende davon verläuft, zwischen den erhabenen Teil und den Führungsteil gedrückt werden, so dass das Spitzenende des Schlauchhauptkörpers in Verbindung mit der Öffnung des äußeren Halteteils gesetzt wird, der Eingriffsteil zum Eingriff zwischen den erhabenen Teil und den Führungsteil ausgelegt ist.

3. Verbindungsstruktur nach Anspruch 1 oder 2, worin der erhabene Teil (15) an dem Umfangsrand der Öffnung in dem äußeren Halteteil geformt ist, und die Länge zwischen dem Abschnitt (52a) des Eingriffsteils, der mit dem Führungsteil in Kontakt ist, und dem Spitzenende (21a) des Schlauchhauptkörpers größer als die Länge zwischen dem erhabenen Teil und dem Führungsteil ist; und ein Verformungsmechanismus bereitgestellt ist, der eine Verformung unter Druck an dem erhabenen Teil gestattet, so dass die Länge zwischen dem erhabenen Teil und dem Führungsteil größer wird, oder ein Verformungsmechanismus bereitgestellt ist, der eine Verformung unter Druck an dem Spitzenende des Schlauchhauptkörpers gestattet, so dass die Länge zwischen dem Abschnitt des Eingriffsteils, der mit dem Führungsteil in Kontakt ist, und dem Spitzenende des Schlauchhauptkörpers kürzer wird.

4. Verbindungsstruktur nach Anspruch 3, worin der Verformungsmechanismus durch Bereitstellung eines Federmechanismus (53) in dem Eingriffsteil ausgelegt ist, um das Spitzenende des Schlauchhauptkörpers bezüglich des Eingriffsteils nach außen zu drücken, und das Spitzenende des Schlauchhauptkörpers durch Aufbringen von Druck darauf veranlasst wird, sich in den Eingriffsteil zurückzuziehen.

5. Verbindungsstruktur nach einem der Ansprüche 1 bis 4, worin der Führungsteil aus einem elastischen Körper besteht und jeweilige Vorsprünge (32a) an beiden Enden des Führungsdrahts auf den Seiten gegenüber dem äußeren Halteteil geformt sind; und wenn der Eingriffsteil zum Eingriff zwischen dem äußeren Halteteil und dem Führungsteil ausgelegt ist, sich der Führungsteil biegt, so dass die Vorsprünge die Endfläche des Eingriffsteils passieren, wonach diese Vorsprünge an der Seite des Eingriffsteils eingreifen.

6. Verbindungsstruktur nach einem der Ansprüche 1 bis 5, worin der Führungsteil und der Verbindungsteil durch ein Verbindungsglied gebildet werden, das von dem äußeren Halteteil getrennt ist, und eine kreisförmige Passkerbe (11c) in der Umfangsrichtung des äußeren Halteteils auf der äußeren Umfangsfläche des äußeren Halteteils geformt ist; und ein Passteil (31) das drehbar in der kreisförmigen Kerbe angebracht ist, in dem Verbindungsteil enthalten ist.

7. Verbindungsstruktur nach einem der Ansprüche 1 bis 6, worin der Gastrostomiekatheter Folgendes umfasst: den äußeren Halteteil; einen schlauchförmigen Teil (12), der so angeordnet ist, dass sein Basisende mit dem äußeren Halteteil verbunden ist und sich im Gebrauch sein Spitzenendabschnitt von der Magenfistel in den Magen-Darm-Trakt erstreckt; und einen inneren Halteteil (13), der mit dem Spitzenende des schlauchförmigen Teils verbunden ist und im Gebrauch in dem Magen-Darm-Trakt angeordnet ist.

8. Verbindungsstruktur nach einem der vorhergehenden Ansprüche, worin der Eingriffsteil (22) zwischen dem äußeren Halteteil (11) und dem Führungsteil (32) in einem Zustand eingreifen kann, in dem das Spitzenende (21a) des Schlauchhauptkörpers (21) in Verbindung mit der Öffnung (14a) des äußeren Halteteils gesetzt wird, indem der Schlauchhauptkörper in den Führungsteil von der offenen Seite her eingeführt wird, so dass er sich zwischen dem äußeren Halteteil und dem Führungsteil aus einer Richtung vorschiebt, die im Wesentlichen senkrecht zu einer Flüssigkeitsdurchgangsrichtung des Gastrostomiekatheters ist.

## Revendications

1. Structure de raccordement pour cathéter de gastrostomie destinée à raccorder un cathéter pour gastrostomie qui est fixé dans une fistule gastrique formée entre la paroi abdominale et le tractus gastro-intestinal d'un patient, la structure de raccordement comportant un cathéter (10) pour gastrostomie, un élément (30) de raccordement et un tube (20) d'alimentation en fluide destiné à être raccordé au cathéter pour gastrostomie afin d'alimenter en fluide le tractus gastro-intestinal du patient dans un passage pour fluide fourni dans le cathéter pour gastrostomie, ladite structure de raccordement étant **caractérisée en ce que** la section du cathéter pour gastrostomie qui, en fonctionnement, est disposée sur la surface de la paroi abdominale au niveau de la fistule gastrique, comporte une partie (11) de retenue extérieure présentant une ouverture (11a) dans le passage pour fluide, **en ce que** l'élément de raccordement comporte une partie (32) guide, dont le bord périphérique interne est sensiblement en forme de C ou en forme de U lorsque vu de dessus, reliée à la partie de retenue extérieure par une partie (33) de liaison, dans un état dans lequel la partie guide est espacée du côté de l'ouverture de la partie de retenue extérieure ; et **en ce qu'**une partie (22) de mise en prise cylindrique est fournie dans la région de l'extrémité de bout d'un corps principal de tube du tube d'alimentation en fluide, ladite partie de mise en prise étant coaxiale avec le corps principal de tube et susceptible de se mettre en prise entre la partie de retenue extérieure et la partie guide dans un état dans lequel l'extrémité de bout du corps principal de tube est placée en communication avec l'ouverture de la partie de retenue extérieure en introduisant le corps principal de tube dans la partie guide depuis son côté ouvert de sorte qu'il avance entre la partie de retenue extérieure et la partie guide.

2. Structure de raccordement selon la revendication 1, dans laquelle la partie guide est constituée d'un corps élastique, et une partie (15) surélevée est formée au niveau du bord périphérique de l'ouverture dans la partie de retenue extérieure ; la longueur entre la section (52a) de la partie de mise en prise qui est en contact avec la partie guide et l'extrémité (21a) de bout du corps principal de tube est supérieure à la longueur entre la partie surélevée et la partie guide, et lorsque la partie de mise en prise et la section du corps principal de tube faisant saillie depuis la partie de mise en prise qui s'étend d'une section dans la région de l'extrémité de bout du corps principal de tube à son extrémité de bout sont enfoncées entre la partie surélevée et la partie guide de sorte que l'extrémité de bout du corps principal de tube est placée en communication avec l'ouverture de la partie de retenue extérieure, la partie de mise en prise est conçue pour être susceptible de se mettre en prise entre la partie surélevée et la partie guide.

3. Structure de raccordement selon la revendication 1 ou 2, dans laquelle la partie (15) surélevée est formée au niveau du bord périphérique de l'ouverture dans la partie de retenue extérieure, et la longueur entre la section (52a) de la partie de mise en prise qui est en contact avec la partie guide et l'extrémité (21a) de bout du corps principal de tube est supérieure à la longueur entre la partie surélevée et la partie guide ; et un mécanisme de déformation est fourni qui permet une déformation lorsqu'une pression est appliquée au niveau de la partie surélevée de sorte que la longueur entre la partie surélevée et la partie guide augmente, ou un mécanisme de déformation est fourni qui permet une déformation lorsqu'une pression est appliquée au niveau de l'extrémité de bout du corps principal de tube de sorte que la longueur entre la section de la partie de mise en prise qui est en contact avec la partie guide et l'extrémité de bout du corps principal de tube diminue.

4. Structure de raccordement selon la revendication 3, dans laquelle le mécanisme de déformation est configuré par la fourniture d'un mécanisme (53) à ressort à l'intérieur de la partie de mise en prise, destiné à pousser l'extrémité de bout du corps principal de tube vers l'extérieur par rapport à la partie de mise en prise, et l'extrémité de bout du corps principal de tube est amenée à se rétracter à l'intérieur de la partie de mise en prise par l'application d'une pression sur cette dernière.

5. Structure de raccordement selon l'une quelconque des revendications 1 à 4, dans laquelle la partie guide est constituée d'un corps élastique, et des saillies (32a) respectives sont formées aux deux extrémités de la partie guide sur les faces opposées à la partie de retenue extérieure ; et lorsque la partie de mise en prise est amenée à se mettre en prise entre la partie de retenue extérieure et la partie guide, la partie guide s'incurve de façon à ce que les saillies se déplacent au-delà de la face d'extrémité de la partie de mise en prise, et les saillies se mettent ensuite en prise du côté de la partie de mise en prise.

6. Structure de raccordement selon l'une quelconque des revendications 1 à 5, dans laquelle la partie guide et la partie de liaison sont constituées d'un élément de raccordement qui est séparé de la partie de retenue extérieure, et une rainure (11c) de montage circulaire est formée dans la direction circonférentielle de la partie de retenue extérieure sur la surface périphérique externe de la partie de retenue extérieure ; et une partie (31) de montage qui est fixée rotative dans la rainure de montage circulaire est comprise dans la partie de liaison.

7. Structure de raccordement selon l'une quelconque des revendications 1 à 6, dans laquelle le cathéter de gastrostomie comporte : la partie de retenue extérieure ; une partie (12) tubulaire qui est disposée de telle sorte que son extrémité de base est jointe à la partie de retenue extérieure et, en fonctionnement, sa section d'extrémité de bout s'étend de la fistule gastrique dans le tractus gastro-intestinal ; et une partie (13) de retenue intérieure qui est jointe à l'extrémité de bout de la partie tubulaire et, en fonctionnement, disposée à l'intérieur du tractus gastro-intestinal.

8. Structure de raccordement selon l'une quelconque des revendications précédentes dans laquelle ladite partie (22) de mise en prise est susceptible de se mettre en prise entre la partie (11) de retenue extérieure et la partie (32) guide dans un état dans lequel l'extrémité (21a) de bout du corps (21) principal du tube est placée en communication avec l'ouverture (14a) de la partie de retenue extérieure en introduisant le corps principal de tube dans la partie guide depuis son côté ouvert de sorte qu'il avance entre la partie de retenue extérieure et la partie guide depuis une direction qui est sensiblement perpendiculaire à la direction du passage pour fluide du cathéter pour gastrostomie.
